# FASCICULE DE BREVET EUROPEEN

(11) **EP 1 435 859 B1**
(45) Date de publication et mention de la délivrance du brevet: **19.01.2005**
(21) Numéro de dépôt: 02785504.8
(22) Date de dépôt: 16.09.2002
(51) Int. Cl.: A61B 17/66, A61B 17/64

(54) **DISTRACTEUR OSSEUX EXTERNE**
EXTERNER KNOCHENDISTRAKTOR
EXTERNAL BONE DISTRACTOR

(30) Priorité: 14.09.2001 FR 0111944
(43) Date de publication de la demande: 14.07.2004
(73) Titulaire: OBL (Société Anonyme), 92320 Chatillon (FR)
(72) Inventeur: LABBE, Daniel, F-14000 Caen (FR); SABIN, Pierre, 76000 Rouen (FR); ROISIN, Jean-Michel, F-91210 Draveil (FR)
(74) Mandataire: Degret, Jacques
(86) Numéro de dépôt international: PCT/FR2002/003156
(87) Numéro de publication internationale: WO 2003/024341

(56) Documents cités:
- EP-A- 0 775 468
- WO-A-99/22661
- US-A- 5 122 140
- US-A- 6 139 316
- PATENT ABSTRACTS OF JAPAN vol. 1997, no. 12, 25 décembre 1997 (1997-12-25) & JP 09 215699 A (NAGOYA RASHI SEISAKUSHO:KK), 19 août 1997 (1997-08-19) cité dans la demande

## Description

Le remplacement d'un fragment osseux manquant à la suite d'une résection tumorale ou d'un traumatisme important fait souvent appel à des techniques opératoires permettant de favoriser la croissance ou le développement de la partie de tissu osseux sain adjacent.

L'une d'entre elles consiste à tirer sur le cal osseux résultant d'une ostéotomie pour l'allonger d'une longueur correspondant à la perte osseuse.

Cette technique, considérablement développée par le praticien russe Ilizarov, était initialement utilisée pour les os longs, ou du moins pour des fragments d'os de forme générale cylindrique.

Le distracteur externe décrit par cet auteur dans le brevet américain US 4.978.348, publié le 18 décembre 1990, semble offrir une souplesse d'utilisation suffisante pour être mis en oeuvre aussi bien pour la restauration de la forme de la main, par étirement des parties de phalanges restantes après amputation, que pour la reconstruction de la mandibule, par exemple.

Le système comporte un ensemble de tiges filetées pouvant être assemblées pour s'adapter à la forme de l'os ou de l'ensemble d'os traités. Les tiges filetées supportent des chariots porte-broches dont les écartements réglables permettent de réaliser la distraction osseuse. Les broches de chacun des chariots sont serrées par un écrou entre des rondelles enfilées sur un boulon creux engagé sur l'une des tiges. Celles-ci présentent un méplat coopérant avec des goujons des boulons creux pour immobiliser les porte-broches en rotation par rapport aux tiges.

Le dispositif est simple, mais les chariots étant maintenus le long des tiges, par des contre-écrous, deux clés sont nécessaires pour les déplacer. De plus, le blocage et le déblocage d'un contre-écrou exigent un maniement simultané relativement complexe de ces deux clés.

Le fixateur externe d'ostéosynthèse divulgué dans la demande de certificat d'utilité FR 2.,671.479 au nom de la société Hit Medica, publiée le 17 juillet 1992, repose sur le principe du ridoir. L'écartement des deux porte-broches est donc simplement obtenu par la manoeuvre de la partie centrale de la tige dont les extrémités sont filetées en sens opposé.

Basé sur un autre principe, le dispositif décrit dans le brevet américain US 4.848.368 publié le 18 juillet 1989 au nom de R. Kronner est quant à lui manoeuvrable par une seule molette. Le patient peut dans ce cas effectuer lui-même l'opération.

Les deux systèmes précédents peuvent être utilisés pour la distraction d'os droits, longs ou courts, mais ils ne conviennent pas pour des os curvilignes comme, par exemple, les os du crâne, les mandibules ou les côtes.

Un dispositif spécialement adapté aux segments osseux courbes des mâchoires, de la face ou du crâne, est décrit dans la demande de brevet allemand DE 195 03 609 publiée le 10 août 1995 au nom de la société Normed Medizin Technik Vertriebs. Le dispositif est formé de plusieurs crémaillères sur lesquelles sont fixés des chariots porte-broches. Les crémaillères sont articulées entre elles pour que l'ensemble épouse extérieurement la forme de l'os. Un chariot peut être déplacé le long d'une crémaillère au moyen d'une vis en prise avec celle-ci.

Les articulations entre les crémaillères comprennent des liaisons sphériques, ce qui présente l'avantage de permettre de donner une forme quelconque à l'ensemble. Cependant, ces articulations ont l'inconvénient majeur de limiter le déplacement des chariots.

Un rail fileté, courbe et continu, ne limitant pas le déplacement des chariots porte-broches, est divulgué dans la demande de brevet japonais JP 09-215699 (Le préambule de la revendication 1 est basé sur ce document) publiée le 19 août 1997 au nom de la Société Nagoya Rashi Seisakusho.

Selon le mode de réalisation représenté sur les figures accompagnant la demande, le rail a une section droite carrée et possède un filetage sur chacune de ses arêtes. Dans l'exemple d'utilisation indiqué, le rail est fixé sur le côté droit d'une mandibule et il suit le contour de celle-ci, d'arrière en avant, sans dépasser l'amorce du menton.

Il semble néanmoins techniquement impossible que le rail décrit dans la demande JP 09-215699 puisse suivre le contour de la mandibule au-delà de l'amorce du menton. Le faible rayon de courbure au niveau du menton aurait pour conséquence évidente de déformer de manière importante les filets de la tige, et d'empêcher la rotation des écrous contrôlant le déplacement des chariots.

En effet, dans la position représentée sur les figures illustrant la demande JP 09-215699, les filets intérieurs à la courbe suivie par la tige sont nécessairement comprimés tandis que les filets extérieurs sont écartés.

Il s'ensuit que le rail courbe semble utilisable seulement pour une réparation d'une partie latérale de la mandibule, comme cela est représenté sur la figure principale, et assurément pas pour une distraction de la partie médiane de la mandibule.

Il ressort donc de l'état de la technique tel que décrit dans les documents cités ci-dessus que des distracteurs osseux externes adaptés à la reconstruction de segments osseux courbes, notamment des os de la mâchoire, de la face et du crâne, sont connus, mais qu'il n'existe à ce jour aucun dispositif de distraction ostéogénique présentant des caractéristiques répondant entièrement aux besoins de la chirurgie maxillo-faciale.

### DESCRIPTION GÉNÉRALE DE L'INVENTION

La présente invention concerne donc un distracteur osseux externe comprenant de manière connue :
- une tige filetée curviligne comportant plusieurs méplats diamétralement opposés préservant le filetage de la tige selon des bandes longitudinales,
- au moins deux chariots engagés sur la tige et solidaires de porte-broches,
- des moyens de guidage coopérant avec lesdits méplats pour bloquer en rotation autour de l'axe de la tige chacun des chariots,
- des moyens de réglage de la position de chacun des chariots le long de la tige.

L'invention a précisément pour objet un distracteur de ce type dont la caractéristique essentielle est que les bandes longitudinales le long desquelles le filetage est préservé s'étendent uniquement au voisinage des fibres neutres de la tige.

Selon une caractéristique additionnelle du distracteur selon l'invention, ces bandes longitudinales sont au nombre de deux seulement et sont diamétralement opposées.

De préférence, la tige forme plus simplement une courbe plane.

Avantageusement, la courbe formée épouse sensiblement la forme de la mandibule. Elle est préférentiellement composée d'un arc de cercle de 150°, dont les extrémités sont prolongées par deux segments de droite.

Dans un mode de réalisation particulier du distracteur, la tige filetée présente six méplats diamétralement opposés, usinés de telle sorte que les côtés de la section droite de la tige, et leurs prolongements, adjacents aux traces des fibres neutres forment un carré.

De préférence, les moyens de guidage comprennent au moins une rondelle solidaire de chacun des chariots engagée sur la tige au moyen d'un alésage axial de section complémentaire de la section droite de ladite tige.

Selon une variante du distracteur osseux externe selon l'invention, ces moyens de guidage comprennent au moins une paire de diabolos d'axes de rotation solidaires de chacun des chariots, agencés à cheval de part et d'autre de la tige et roulant sur celle-ci.

Quant aux moyens de réglage, ils comprennent de préférence un écrou moleté vissé sur la tige agissant sur chacun des chariots au moyen d'un étrier.

Avantageusement, les moyens de guidage d'un chariot comportent deux positions : la direction du porte-broche dans l'une des positions est perpendiculaire à la direction du porte-broche dans l'autre position.

On tirera bénéfice du fait qu'au moins deux des chariots du distracteur osseux externe selon l'invention sont fixes, et qu'un troisième au moins est mobile le long de la tige.

### BRÈVE DESCRIPTION DES DESSINS

Les Figures 1, 3 et 4 montrent respectivement une vue schématique de dessous, de face et de gauche, du distracteur osseux selon l'invention.
La Figure 2 montre une section droite agrandie de la tige filetée que comporte le distracteur selon un mode de réalisation préféré de l'invention.
Les Figures 5a et 5b montrent respectivement une vue de dessus et une vue de face d'un premier mode de réalisation de l'un des chariots porte-broche du distracteur selon l'invention.
Les Figures 6a et 6b montrent respectivement une vue de dessus et une vue de face d'une variante de réalisation de l'un des chariots porte-broche, dont le couvercle a été enlevé, du distracteur selon l'invention.

### DESCRIPTION DES FORMES D'EXÉCUTION PRÉFÉRÉES DE L'INVENTION

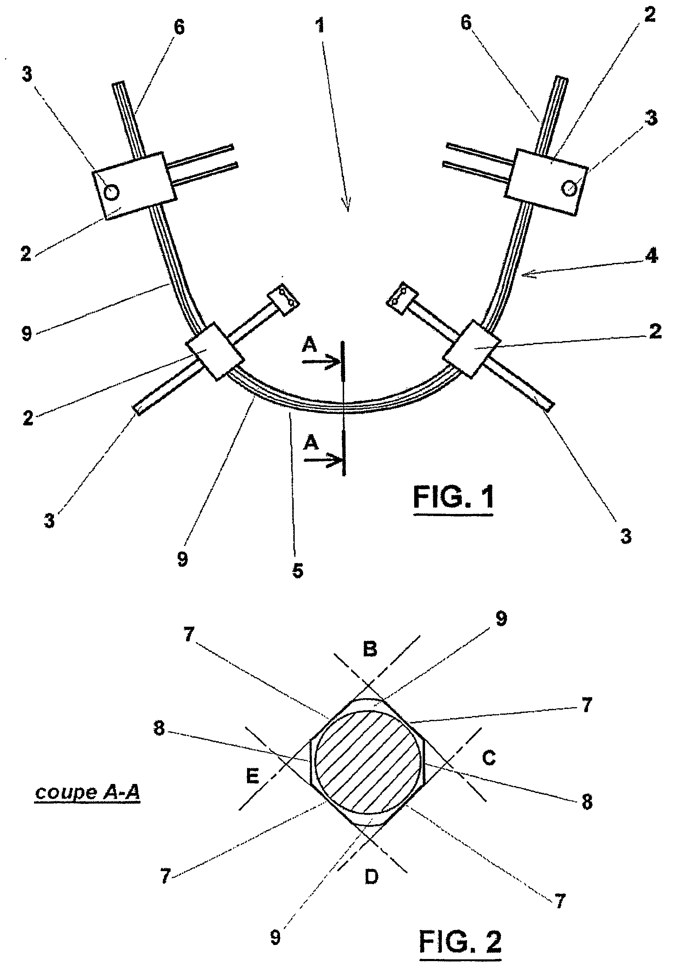

Les références à la Figure 1 et à la Figure 2 serviront à expliquer le principe général de l'invention en s'appuyant sur un modèle d'appareil adapté à la reconstruction mandibulaire par distraction osseuse progressive.

La Figure 1 représente de façon simplifiée une vue de dessous du distracteur 1. On reconnaît des chariots 2 supportant des porte-broches 3 engagés sur une tige filetée 4.

A la différence des dispositifs connus, comportant une ou plusieurs tiges rectilignes, cette tige filetée 4 est continue et sa forme épouse extérieurement sensiblement la forme de la mandibule.

Elle comprend une section avant 5 en forme d'arc de cercle de 150° et d'un rayon de 70 mm, ainsi que deux sections latérales 6 constituées de segments rectilignes de 90 mm de long.

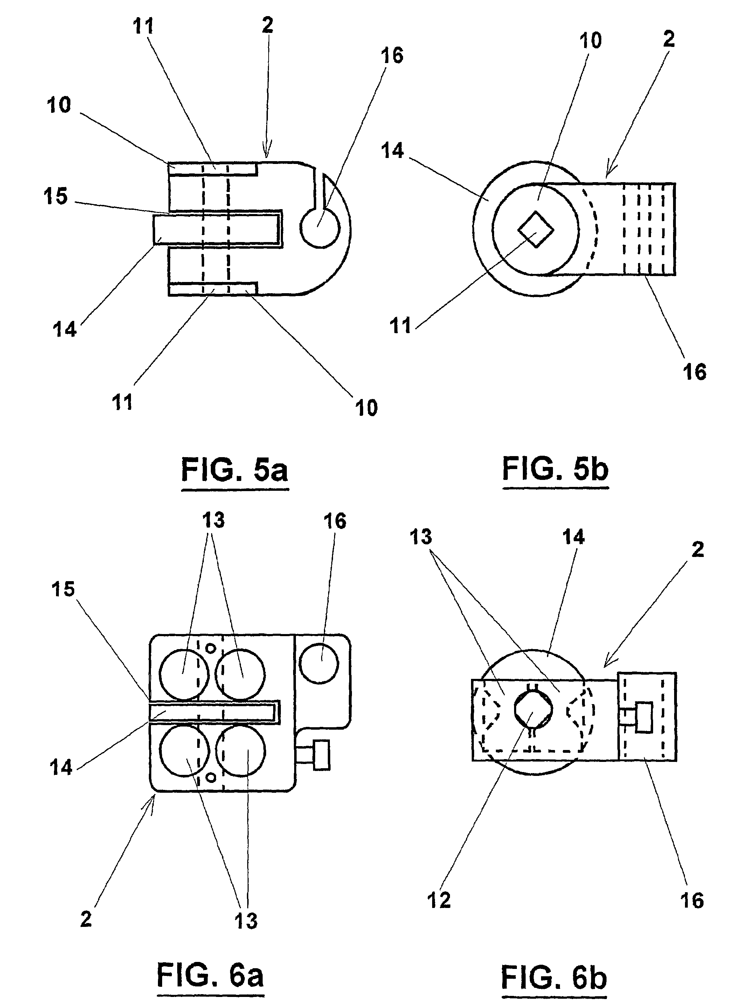

La Figure 2 montre bien en coupe (Coupe AA) l'autre caractéristique essentielle de la tige filetée 4. Six méplats 7,8 pris dans une tige filetée au standard ISO M6 ne préservent le filetage 9 qu'au voisinage des fibres neutres de la tige 4, c'est-à-dire les fibres de la tige 4 qui ne subissent ni extension, ni compression et qui sont donc situées au voisinage de chacun des deux plans tangentiels à la tige 4 qui sont parallèles au plan dans lequel ladite tige s'étend. Chaque partie préservée 9 du filetage conserve donc les caractéristiques nominales du profil des filets et du pas de la tige filetée 4 et peut par conséquent coopérer avec un écrou de la dimension correspondante. Cet écrou, comme il le sera expliqué en liaison avec les Figures 5 et 6, constituera un moyen de réglage de la position d'un chariot 2 le long de la tige 4.

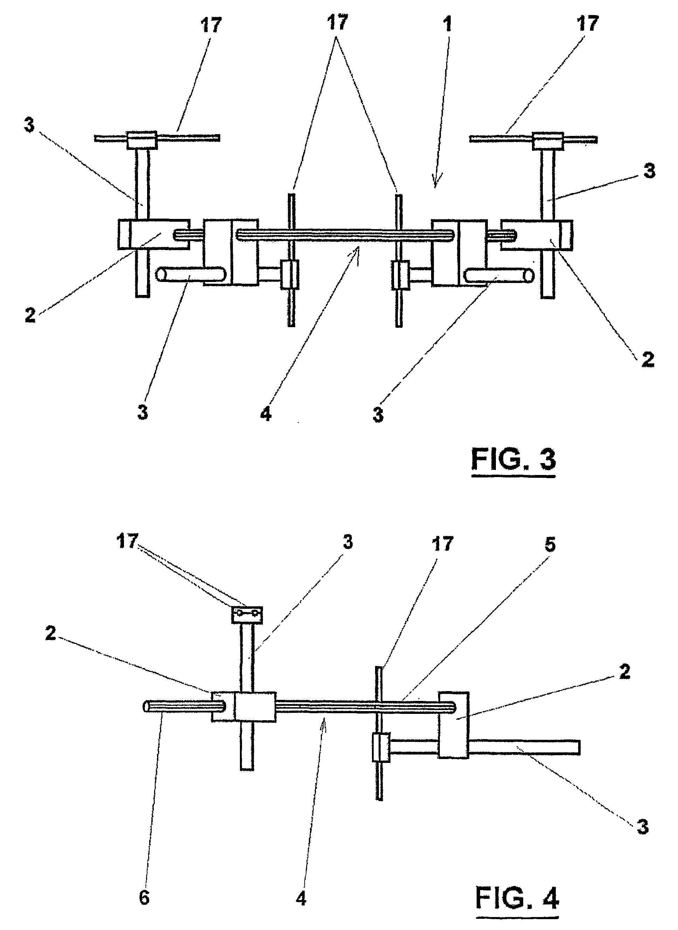

Comme le montrent les Figures 3 et 4, les chariots 2 sont capables de maintenir les porte-broches 3 soit dans une position perpendiculaire au plan du distracteur 1 (chariots 2 sur la section avant 5), soit dans une position parallèle (chariots 2 sur les sections latérales 6).

Pour ce faire, les chariots 2 sont guidés sur la tige 4 et bloqués en rotation sur celle-ci par des moyens de guidage adaptés à la forme particulière de la section (Figure 2) de la tige filetée 4.

Les côtés 7 de cette section droite adjacents aux parties préservées 9 du filetage au voisinage des fibres neutres, et leurs prolongements, forment en effet un carré BCDE.

Dans un premier mode de réalisation préféré du distracteur 1, un chariot 2 est solidaire de deux rondelles 10 alignées dont l'alésage axial 11 est de section carrée, comme le montre bien la Figure 5b, c'est-à-dire complémentaire de la tige 4 sur laquelle elles sont engagées. Le chariot 2 peut donc coulisser sur la tige filetée 4, mais il ne présente que deux positions en rotation axiale, à 90° l'une de l'autre, selon la façon dont il a été enfilé sur la tige 4.

Selon une variante de réalisation représentée sur les Figures 6a et 6b, le canal 12 de section carrée dans lequel circule la tige 4 filetée à l'intérieur d'un chariot 2 est obtenu au moyen de deux paires de diabolos 13 alignées. Les diabolos 13 tourillonnent dans quatre logements cylindriques du massif constituant le chariot 2, ce qui permet à la tige filetée 4 de coulisser tout en étant guidée.

Le chariot est représenté "couvercle enlevé", de façon à rendre visible ces diabolos 13 sur la Figure 6a.

Comme précédemment, le chariot 2 peut être orienté sur la tige de deux manières différentes.

L'espace libre laissé dans le massif du chariot 2, entre les rondelles 10 ou les diabolos 13 pour accueillir un écrou moleté 14 vissé sur la tige 4, forme un étrier 15 transmettant au chariot 2 les déplacements de cet écrou 14.

Celui-ci peut être facilement manoeuvré par le patient lui-même. Un cliquet rend sensible le nombre de fractions de tour effectué.

Des alésages cylindriques 16 sont prévus dans les massifs des chariots pour recevoir les tiges des porte-broches 3, qui sont immobilisées par des vis.

Ces structures très légères et très simples de chariots 2 permettent donc, conformément aux vues du distracteur 1 selon l'invention des Figures 1, 3 et 4, de placer idéalement les broches 17 aux endroits les plus propices de la mandibule pour mener à bien une distraction osseuse sans incident.

Le profil du chemin des chariots 2, adapté au mieux à la forme de la mandibule, rend la distraction particulièrement progressive.

On conçoit que la tige 4 servant de rail doive être maintenue solidaire de la mandibule par l'intermédiaire des broches de deux chariots 2 fixes au minimum. Un troisième chariot au moins est mobile pour effectuer le transport d'os.

Les avantages de la forme courbe de la tige filetée 4 sont aisément transposables à d'autres cas que celui de la mandibule; une tige de dimensions et de profil adaptés à ceux du crâne constituerait la base d'un appareillage de distraction de la boîte crânienne.

Comme il va de soi, l'invention ne se limite pas aux seules spécifications techniques ci-dessus, données à titre d'exemple; elle embrasse, au contraire, toutes les autres variantes possibles de réalisation.

En particulier, les moyens de guidage 10,13 des chariots 2 ou de réglage de leurs positions 14 exposés ci-dessus ne sont pas limitatifs. Tout autre moyen mécanique mis en oeuvre aux mêmes fins est une variante possible qui ne sort pas du concept inventif du distracteur osseux externe dont les caractéristiques sont spécifiées ci-dessous.

## Revendications

1. Distracteur osseux externe (1) comprenant:
- une tige curviligne filetée (4) comportant plusieurs méplats (7,8) diamétralement opposés préservant le filetage (9) de ladite tige (4) selon des bandes longitudinales,
- au moins deux chariots (2) engagés sur ladite tige (4) et solidaires de porte-broches (3),
- des moyens de guidage (10,13) coopérant avec lesdits méplats (7,8) pour bloquer en rotation autour de l'axe de ladite tige (4) chacun desdits chariots (2),
- des moyens de réglage (14,15) de la position de chacun desdits chariots (2) le long de ladite tige (4),
**caractérisé en ce que** lesdites bandes s'étendent uniquement au voisinage des fibres neutres de ladite tige (4).

2. Distracteur osseux externe (1) selon la revendication 1, **caractérisé en ce que** les bandes (9) sont au nombre de deux seulement et sont diamétralement opposées.

3. Distracteur osseux externe (1) selon l'une quelconque des revendications 1 ou 2 précédentes, **caractérisé en ce que** la tige (4) forme une courbe plane.

4. Distracteur osseux externe (1) selon la revendication 3, **caractérisé en ce que** la tige (4) forme une courbe épousant extérieurement sensiblement la forme de la mandibule, préférentiellement composée d'un arc de cercle (5) de 150 °, dont les extrémités sont prolongées par deux segments de droite (6).

5. Distracteur osseux externe (1) selon la revendication 4, **caractérisé en ce que** la tige (4) présente six méplats (7,8) diamétralement opposés, usinés de telle sorte que les côtés de la section droite (AA) de ladite tige (4), et leurs prolongements, adjacents aux traces des fibres neutres (9) forment un carré (BCDE).

6. Distracteur osseux externe (1) selon l'une quelconque des revendications 1 à 5 précédentes, **caractérisé en ce que** les moyens de guidage (10,13) comprennent au moins une rondelle (10) solidaire de chacun des chariots (2), engagée sur la tige (4) au moyen d'un alésage axial (11) de section complémentaire de la section droite (AA) de ladite tige (4).

7. Distracteur osseux externe (1) selon la revendication 5, **caractérisé en ce que** les moyens de guidage (10,13) comprennent au moins une paire de diabolos (13) d'axes de rotation solidaires de chacun des chariots (2), agencés à cheval de part et d'autre de la tige (4), et roulant sur celle-ci.

8. Distracteur osseux externe (1) selon l'une quelconque des revendications 1 à 7 précédentes, **caractérisé en ce que** les moyens de réglage (14,15) comprennent un écrou moleté (14) vissé sur la tige (4) agissant sur chacun des chariots (2) par l'intermédiaire d'un étrier (15).

9. Distracteur osseux externe (1) selon l'une quelconque des revendications 1 à 8 précédentes, **caractérisé en ce que** chacun des moyens de guidage (10,13) comporte deux positions, la direction du porte-broche (3) dans l'une desdites positions étant perpendiculaire à la direction du porte-broche (3) dans l'autre desdites positions.

10. Distracteur osseux externe (1) selon l'une quelconque des revendications 1 à 9 précédentes, **caractérisé en ce qu'**au moins deux desdits chariots (2) sont fixes, et qu'un troisième au moins est mobile le long de la tige (4).

## Patentansprüche

1. Externer Knochendistraktor (1), mit:
- einer gekrümmten Gewindestange (4), die mehrere Abflachungen (7, 8) aufweist, die diametral gegenüberliegend angeordnet sind, wobei sie das Gewinde (9) der Stange (4) entlang von Längsbahnen schützen,
- wenigstens zwei Schlitten (2), die in Eingriff mit der Stange (4) sind und mit Stiftträgern (3) verbunden sind,
- Führungseinrichtungen (10, 13), die mit den Abflachungen (7, 8) zusammenwirken, um jeden der Schlitten (2) an einer Drehung um die Achse der Stange (4) zu hindern,
- Einstelleinrichtungen (14, 15) der Position eines jeden der Schlitten (2) entlang der Stange (4),
**dadurch gekennzeichnet, dass** die Bahnen sich nur in der Nähe der neutralen Fasern der Stange (4) erstrecken.

2. Externer Knochendistraktor (1) nach Anspruch 1, **dadurch gekennzeichnet, dass** die Bahnen (9) nur in einer Anzahl von zwei vorhanden und diametral entgegengesetzt angeordnet sind.

3. Externer Knochendistraktor (1) nach Anspruch 1 oder 2, **dadurch gekennzeichnet, dass** die Stange (4) eine ebene Krümmung bildet.

4. Externer Knochendistraktor (1) nach Anspruch 3, **dadurch gekennzeichnet, dass** die Stange (4) eine Krümmung bildet, die aussen im wesentlichen der Form der Mandibule folgt, vorzugsweise gebildet aus einem Kreisbogen (5) von 150 °, dessen Enden durch zwei gerade Segmente (6) verlängert sind.

5. Externer Knochendistraktor (1) nach Anspruch 4, **dadurch gekennzeichnet, dass** die Stange (4) sechs diametral entgegengesetzt angeordnete Abflachungen (7, 8) aufweist, die derart ausgebildet sind, dass die Seiten des Querschnitts (AA) der Stange und ihre zu den Bahnen der neutralen Fasern (9) benachbarten Verlängerungen ein Quadrat (BCDE) bilden.

6. Externer Knochendistraktor (1) nach einem der vorhergehenden Ansprüche 1 bis 5, **dadurch gekennzeichnet, dass** die Führungseinrichtungen (10, 13) wenigstens eine Scheibe (10) aufweisen, die mit einem jeden der Schlitten (2) verbunden ist, in Eingriff mit der Stange (4) mit Hilfe einer axialen Ausnehmung (11) von komplementären Querschnitt zum Querschnitt (AA) der Stange (4).

7. Externer Knochendistraktor (1) nach Anspruch 5, **dadurch gekennzeichnet, dass** die Führungseinrichtungen (10, 13) wenigstens ein Paar von doppelkegeligen Rollen (13) aufweisen, deren Drehachsen mit einem jeden der Schlitten (2) verbunden sind, wobei sie beidseitig an der Stange (4) angeordnet und auf dieser rollend ausgebildet sind.

8. Externer Knochendistraktor (1) nach einem der vorhergehenden Ansprüche 1 bis 7, **dadurch gekennzeichnet, dass** die Einstelleinrichtungen (14, 15) eine Rändelmutter (14) aufweisen, die auf die Stange (4) aufgeschraubt ist, wobei sie auf einen jeden der Schlitten (2) mittels eines Bügels (15) wirkt.

9. Externer Knochendistraktor (1) nach einem der Ansprüche 1 bis 8, **dadurch gekennzeichnet, dass** eine jede der Führungseinrichtungen (10, 13) zwei Positionen aufweist, wobei die Richtung des Stiftträgers (3) in einer der Positionen senkrecht verläuft zur Richtung des Stiftträgers (3) in der anderen der Positionen.

10. Externer Knochendistraktor (1) nach einem der Ansprüche 1 bis 9, **dadurch gekennzeichnet, dass** wenigstens zwei Schlitten (2) fest sind und dass wenigstens ein dritter entlang der Stange (4) beweglich ist.

## Claims

1. An external bone distractor (1) comprising:
- a threaded curvilinear rod (4) comprising a plurality of diametrically opposed flat sections (7, 8), preserving the threading (9) of the rod (4) along longitudinal bands,
- at least two carriages (2) engaging on said rod (4) and solidly attached to pin supports (3),
- guide means (10, 13) which co-operate with said flat sections (7, 8) in order to prevent the rotation of each of said carriages (2) around the axis of said rod (4),
- means (14, 15) for adjusting the position of each of said carriages (2) along said rod (4),
**characterised in that** said bands extend solely in the vicinity of the neutral fibres of said rod (4).

2. An external bone distractor (1) according to claim 1, **characterised in that** there are only two bands (9) and they are diametrically opposed.

3. An external bone distractor (1) according to any one of the preceding claims 1 or 2, **characterised in that** the rod (4) forms a flat curve.

4. An external bone distractor (1) according to claim 3, **characterised in that** the rod (4) forms a curve which substantially fits the shape of the jaw on the outside, preferably made up of a 150° arc of a circle (5), the ends of which are extended by two straight segments (6).

5. An external bone distractor (1) according to claim 4, **characterised in that** the rod (4) has six diametrically opposed flat sections (7, 8) machined so that the sides of the straight section (AA) of said rod (4), and their extensions, adjacent to the lines of the neutral fibres (9), form a square (BCDE).

6. An external bone distractor (1) according to any one of the preceding claims 1 to 5, **characterised in that** the guide means (10, 13) comprise at least one washer (10) solidly attached to each of the carriages (2), engaging on the rod (4) by means of an axial bore (11) of complementary section to the straight section (AA) of said rod (4).

7. An external bone distractor (1) according to claim 5, **characterised in that** the guide means (10, 13) comprise at least one pair of dual wheels (13) the rotation axles of which are integral with those of the carriages (2), arranged so as to straddle the rod (4) on either side, and rolling thereon.

8. An external bone distractor (1) according to any one of the preceding claims 1 to 7, **characterised in that** the adjusting means (14, 15) comprise a knurled nut (14) screwed onto the rod (4) acting on each of the carriages (2) via a bracket (15).

9. An external bone distractor (1) according to any one of the preceding claims 1 to 8, **characterised in that** each of the guide means (10, 13) has two positions, the direction of the pin support (3) in one of said positions being perpendicular to the direction of the pin support (3) in the other said position.

10. An external bone distractor (1) according to any one of the preceding claims 1 to 9, **characterised in that** at least two of said carriages (2) are fixed, and at least a third is movable along the rod (4).
